# EUROPEAN PATENT SPECIFICATION

(11) **EP 3 568 726 B1**
(45) Date of publication and mention of the grant of the patent: **06.03.2024**
(21) Application number: 18739217.0
(22) Date of filing: 12.01.2018
(51) Int. Cl.: G02C 9/00, G02C 5/12, A61F 9/02

(54) **FRAME SUPPORT MEMBER AND FRAME SUPPORT ASSEMBLY FOR OVER-THE-GLASSES (OTG) EYEWEAR**
RAHMENSTÜTZGLIED UND RAHMENSTÜTZANORDNUNG FÜR OVER-THE-GLASSES (OTG)-BRILLE
ÉLÉMENT DE SUPPORT DE MONTURE ET ENSEMBLE DE SUPPORT DE MONTURE POUR LUNETTES DU TYPE OVER-THE-GLASSES (OTG)

(30) Priority: 12.01.2017 US 201715404943
(43) Date of publication of application: 20.11.2019
(73) Proprietor: Sheldon, Brent, Miami Beach, Florida 33139 (US)
(72) Inventor: BROUSSEAU, JR., Guy, Marieville Québec J3M 0A5 (CA); LAPERLE, Amélie Brisson, Saint-Basile-Le-Grand Québec J3N 1S9 (CA); ORBAN, Benoit, Saint-Lambert Québec J4P 2K1 (CA); LÉVEILLÉ, Manuel, Montréal Québec H1X 3K2 (CA); SHELDON, Brent, Montréal Québec H1X 3K2 (US)
(74) Representative: Ribeiro, James Michael
(86) International application number: PCT/CA2018/050029
(87) International publication number: WO 2018/129623

(56) References cited:
- WO-A2-2012/005509
- FR-A1- 2 818 394
- US-A- 2 607 919
- US-A- 5 971 538
- US-A1- 2002 101 561
- US-A1- 2012 147 317
- US-A1- 2012 206 690
- US-A1- 2014 375 948
- US-B1- 7 591 555
- US-B2- 7 204 589
- US-B2- 7 497 570

## Description

### CROSS-REFERENCE TO RELATED APPLICATION(S)

This application claims priority to U.S. Application No. 15/404,943 filed on January 12, 2017.

### TECHNICAL FIELD

The following relates to support assemblies for eyewear, in particular to position secondary eyewear such as over-the-glasses (OTG) eyewear, relative to primary eyewear, such as prescription eyewear.

### DESCRIPTION OF THE RELATED ART

Conventional eyewear, particularly prescription eyeglasses, may be required by a user at all or most times to improve their vision. Eyeglasses generally include a frame that supports one or more lenses. The frame typically includes a nose bridge or nose pieces that engage the user's nose to support the eyeglasses on the user's head. Eyeglasses also typically include a pair of arms attached to (or integral with) the frame, to further support the eyeglasses, *e.g.* by resting the arms on the user's ears or engaging their head in the temple region.

Safety eyewear is required in various scenarios, such as on a job or construction site, shop or factory floor, etc. While prescription safety eyewear exists, the costs associated with having prescription lenses for safety eyewear can be prohibitive. Moreover, having prescription lenses for safety eyewear may not be feasible, particularly when the safety eyewear is meant to be shared and reused, *e.g.,* by visitors to a jobsite. For these reasons, safety eyewear has been known to be constructed to fit over a conventional pair of eyeglasses in order to eliminate the need for the user to remove their prescription eyewear or to possess prescription safety eyewear. Such solutions are commonly referred to as "over the glasses" or "OTG" type eyewear.

One problem with OTG type eyewear is that the OTG frames often rest on the prescription frames, which can cause the prescription frames to be pulled downwardly on the user's nose, causing misalignment of the lenses and/or causing general discomfort.

Moreover, typical OTG frames may rest or be pushed against the front of the prescription lenses, which can cause damage to the more valuable eyewear, or cause the prescription lenses to impinge the user's face This may occur when the OTG frames act as they are intended to, namely to deflect debris and other objects from the user's face.
US5971538 discloses an articulated nose bridge for a head mounted display for adjusting the height, tilt, and distance of the display on the wearer's face. The articulated nose bridge includes a nose piece configured to rest on a wearer's nose, a bridge piece located between a left eye display and a right eye display of the head mounted display, and an articulator. The articulator includes a nose end coupled to the nose piece, and a bridge end pivotally coupled to the bridge piece and allowing a viewing angle of the left eye display and the right eye display to be adjusted by the wearer. In addition, the nose end of the articulator is pivotally coupled to the nose piece.
US2002/101561 discloses an eyeglass frame with an adjustable nose piece assembly for adjusting the eyeglasses to the nose and face of a wearer. The nose piece assembly is readily removable from a bracket secured between two lens receiving sections for replacement purposes or for storing in a flattened condition. A pair of temples are hingedly mounted to the lens receiving sections to be folded into a flattened condition.
US7591555 discloses a framework of combination of spectacle frames with lenses comprises a spectacle frame provided with an upper arm and assembled with a nosepiece, in which a hole seat is formed between the upper arm and the nosepiece and a wedge hole is formed inside the hole seat; a lens provided with a neck, in which adjustment of multiple segments may be made for clamping by using a gibbous wedge of the nosepiece that is arranged in the wedge hole, and a distance between the gibbous wedge and the upper arm may be adjusted for the tight fitness of lens.

It is an object of the following to address at least one of the above-noted disadvantages.

### SUMMARY

In order to support secondary eyewear (*e.g.,* safety eyewear) that is worn over primary eyewear (*e.g.,* prescription eyewear), a frame support member is provided that engages the user's nose to support the secondary eyewear above and away from the primary eyewear frames and thus reduce interference between the primary and secondary eyewear frames. The support member can also reduce or eliminate downward forces imparted by the secondary eyewear on the primary eyewear by having at least a portion of a nose-engaging portion be positioned below and behind the lenses of the secondary eyewear such that the secondary eyewear is physically separated from the underlying primary eyewear. Separation can also be provided between the secondary eyewear and the lenses of the primary eyewear by incorporating the support member. In some implementations, a frame support assembly is provided that includes the support member with adjustability. The assembly or support member can be adjusted through flexure or extendibility to accommodate different users. The assembly or support member, and/or aspects thereof can also be used for primary eyewear that is worn on its own (*e.g.,* safety eyewear worn without underlying prescription eyewear).

In one aspect, there is provided a frame support member for eyewear according to claim 1.

In another aspect, there is provided eyewear according to claim 7.

In yet another aspect, there is provided a frame support assembly for eyewear comprising a frame, the frame of the eyewear comprising a nose bridge, the frame support assembly comprising: a frame support member according to claim 1 comprising an attachment portion at a first end thereof for coupling the frame support member to the nose bridge of the eyewear, and a nose engaging portion at a second end thereof, wherein the frame support member is configured to position the nose engaging portion below and behind the nose bridge for supporting the eyewear on a user; and an extender coupled to the frame support member at one end and a frame engaging portion at the other end, the frame engaging portion configured to be coupled to the nose bridge of the frame.

In yet another aspect, there is provided a frame support member for eyewear according to claim 1 comprising a frame and a lens, the frame of the eyewear comprising a nose bridge portion, the frame support member comprising: an extendable attachment portion for coupling the frame support member to the nose bridge portion of the eyewear, wherein the attachment portion comprises a contoured aperture for receiving a post extending from the nose bridge portion or the lens of the eyewear, the post engaging the contoured aperture to provide a plurality of vertical positions relative to the frame; and a nose engaging portion extending from the attachment portion for supporting the eyewear on a user.

In yet another aspect, there is provided eyewear according to claim 7 comprising: a frame for supporting at least one lens, the frame comprising nose bridge portion; a frame support member comprising an extendable attachment portion for coupling the frame support member to the nose bridge portion of the eyewear, wherein the attachment portion comprises a contoured aperture for receiving a post extending from the nose bridge portion or the lens of the eyewear, the post engaging the contoured aperture to provide a plurality of vertical positions relative to the frame; and a nose engaging portion extending from the attachment portion for supporting the eyewear on a user; wherein the frame support member is configured to position the nose engaging portion below and behind the nose bridge for supporting the eyewear on a user.

### BRIEF DESCRIPTION OF THE DRAWINGS

Embodiments will now be described by way of example only with reference to the appended drawings wherein:
FIG. 1 is a perspective view of a user with over the glasses (OTG) eyewear worn over a prescription eyewear frame;
FIG. 2 is a side view of a user with OTG eyewear worn over a prescription eyewear frame;
FIG. 3 is a perspective view of only the OTG eyewear worn by the user;
FIG. 4 is a perspective view of OTG eyewear showing a rear view of a frame support member;
FIG. 5 is a partial enlarged rear perspective view of the frame support member;
FIG. 6 is a side view of a frame support member in isolation;
FIG. 7(a) is a front view of the frame support member in one implementation;
FIG. 7(b) is a front view of the frame support member in an example not covered by the invention having a contoured nose-engaging portion;
FIG. 8(a) is a side view of an alternative frame support member configuration;
FIG. 8(b) is a side view of yet another alternative frame support member configuration;
FIGS. 9(a) to 9(c) illustrate perspective views of a frame support assembly with an adjustable frame support member in a series of positions;
FIGS. 10(a) to 10(d) are schematic views illustrating adjustability features for a frame support assembly with adjustable and flexible frame support members;
FIG. 11 is a side view of yet another alternative frame support member configuration having extendibility in multiple directions;
FIG. 12 is a rear view of a ratchet-type extender for a frame support assembly;
FIG. 13 is a front perspective view of an eyewear frame assembly having yet another alternative frame support member according to an example not covered by the invention interposed between a frame portion and a lens portion;
FIG. 14 is a rear perspective view of the frame support member of FIG. 13.
FIG. 15(a) is a perspective view of the frame support member supported on a post protruding from the frame portion;
FIG. 15(b) is a perspective view of the frame support member assembled with the frame portion and the lens portion;
FIG. 16 is a rear perspective view of the assembly shown in FIG. 13;
FIG. 17 is a sectional view of the assembled frame support member;
FIG. 18 is a front view of the assembled frame support member;
FIG. 19 is a sectional side view of the frame support member along section A-A in FIG. 18;
FIG. 20 is a sectional side view of the frame support member in an alternative position;
FIG. 21 is a sectional side view of the frame support member in another alternative position;
FIG. 22 is a front perspective view of an eyewear frame assembly having yet another alternative frame support member interposed between a frame portion and a lens portion;
FIG. 23 is a rear perspective view of the eyewear frame assembly of FIG. 22;
FIG. 24 is an exploded rear perspective view of the eyewear frame assembly of FIG. 22;
FIG. 25 is a sectional view of a nose bridge portion and the frame support member of FIG. 23 along line B-B;
FIG. 26 is a sectional view along the line C-C in FIG. 23; and
FIGS. 27(a), 27(b), and 27(c) are rear perspective views of low, mid and high positions for the frame support member of FIG. 22.

### DETAILED DESCRIPTION

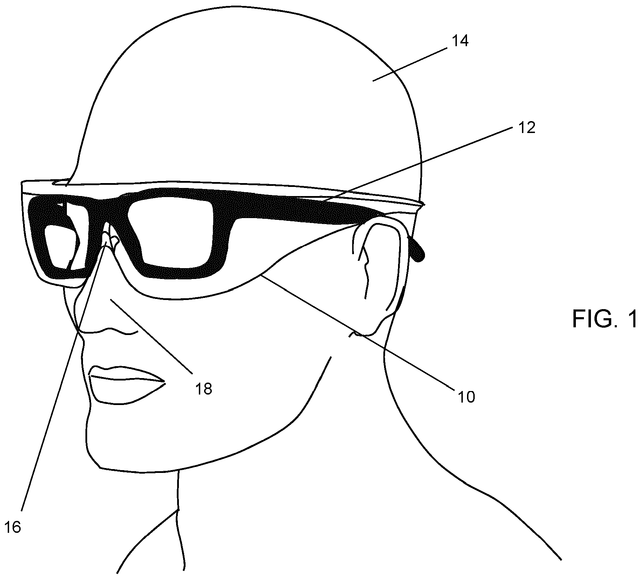

Turning now to the figures, FIG. 1 illustrates safety eyewear 10 (i.e. "secondary" eyewear) being worn over an underlying eyewear (i.e., "primary" eyewear), in this example a pair of prescription eyeglasses 12. Both the safety eyewear 10 and the underlying prescription eyeglasses 12 are shown as being worn by a user 14 and are supported on the user 14 at least in part by resting on the user's nose 18. The safety eyewear 10 is supported relative to the prescription eyeglasses 12 using a frame support member 16 that engages the user's nose 18 as explained in greater detail below. A frame support assembly that incorporates the frame support member 16 and other features providing adjustability may also be provided, as also explained in greater detail below.

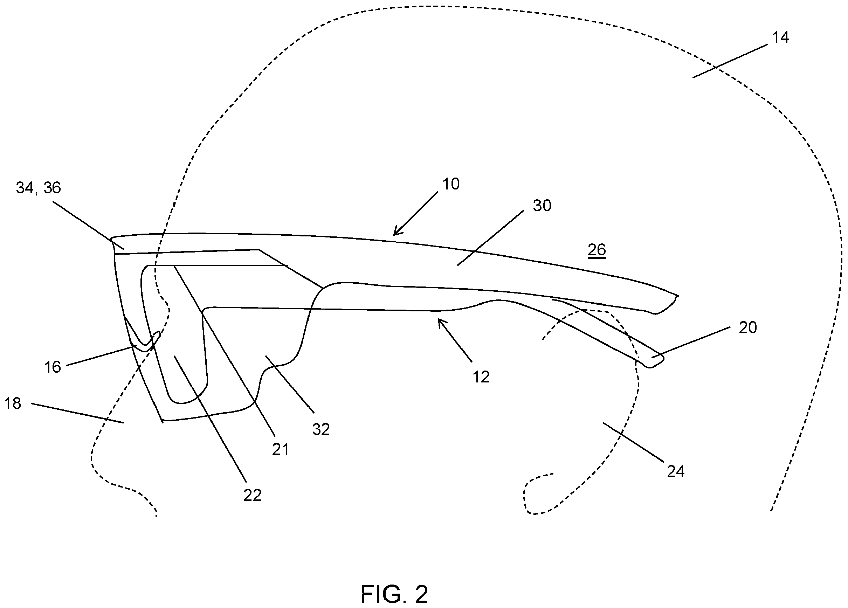

As illustrated in FIG. 2, the prescription eyeglasses 12 include a pair of arms 20 that are either pivotally attached to, or integral with a frame 21 that holds or otherwise supports a pair of prescription lenses 22 as is known in the art. The prescription eyeglasses 12 can also be further supported on the user 14 by resting the arms 20 on the user's ears 24. The safety eyewear 10 in the example shown in FIG. 2 includes a pair of arms 30 that are either pivotally attached to, or integral with a frame 34 that holds or otherwise supports a pair of safety lenses 32. The safety eyewear 10 in the example shown in FIG. 2 are additionally supported on the user by engaging temple portions 26 of the user's head 14. However, it can be appreciated that the arms 30 of the safety eyewear 10 can instead rest upon the user's ears 24. The safety lenses 32 can be made from any suitable material providing impact and shatter resistance and, as illustrated in FIG. 2, these lenses 32 can partially wrap around the user's head 14 to provide side impact protection.

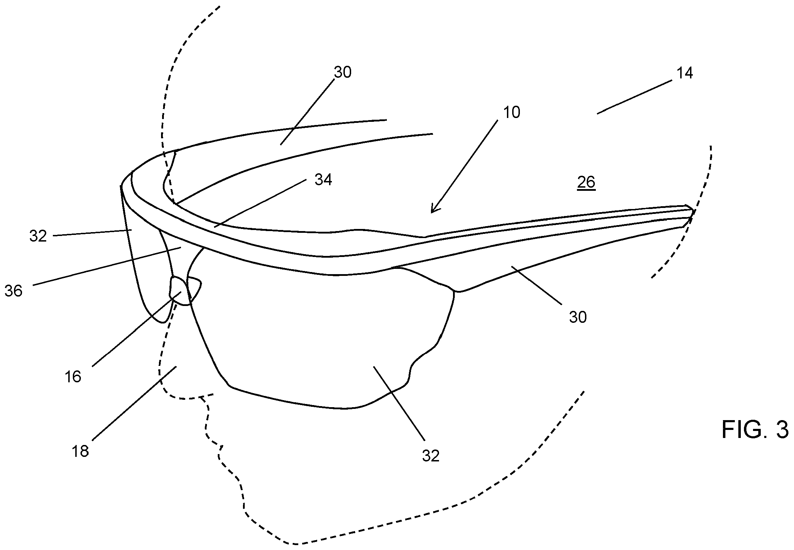

Referring to both FIGS. 2 and 3, the frame 34 can include a nose bridge 36 that is positioned between the pair of lenses 32. It can be appreciated that the nose bridge 36 can also be formed from a central portion of a single lens 32 and need not be a separate element. As can be seen in FIG. 2, the frame support member 16 is shaped such that it extends downwardly and rearwardly (i.e. below and behind the lenses 32) to engage the user's nose 18. In this way, the safety eyewear 10 is separately and independently supported on the user's nose 18 by being positioned above the frame 21 and away from the lenses 22 of the primary eyewear. That is, in this example, the frame support member 16 inhibits the safety eyewear 10 from resting upon the underlying prescription eyeglasses 12 which, as explained above, can cause the safety eyewear 10 to pull or drag the prescription eyeglasses 12 down the user's nose and cause misalignment of the lenses 22 with the user's eyes and/or general discomfort.

By providing inherent adjustability in the frame support member 16 itself, or by providing a frame support assembly that provides adjustability with/for the frame support member 16, the frame support member 16 can enable the same pair of safety eyewear 10 to be adjusted to suit different users with different head sizes, shapes, positioning of facial features, etc.

The view shown in FIG. 3 also illustrates that the frame support member 16 is also suitable for use with any eyewear, including primary eyewear without any secondary eyewear (or vice versa). While FIG. 3 shows the frame support member 16 used with safety eyewear 10, it can be appreciated that the frame support member 16 can also be used with prescription eyeglasses 12 or any other type of eyewear such as sports eyewear or an eyewear accessory (*e.g.,* a magnetic or other type of clip-on sunglasses).

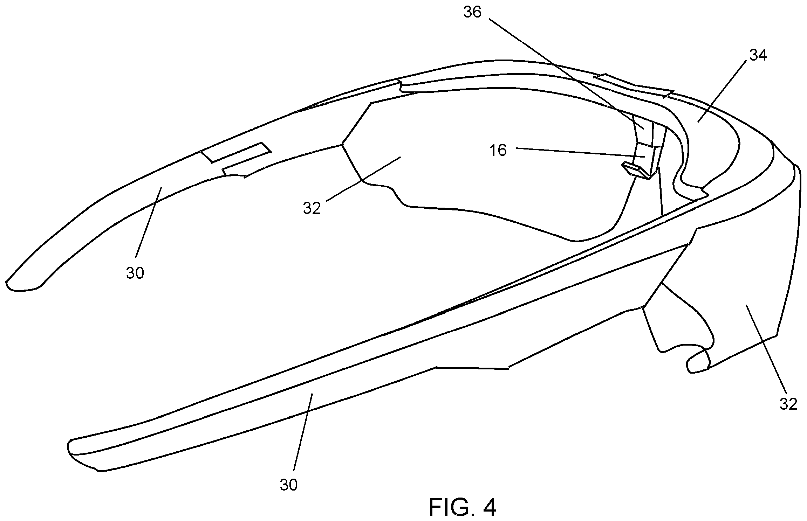

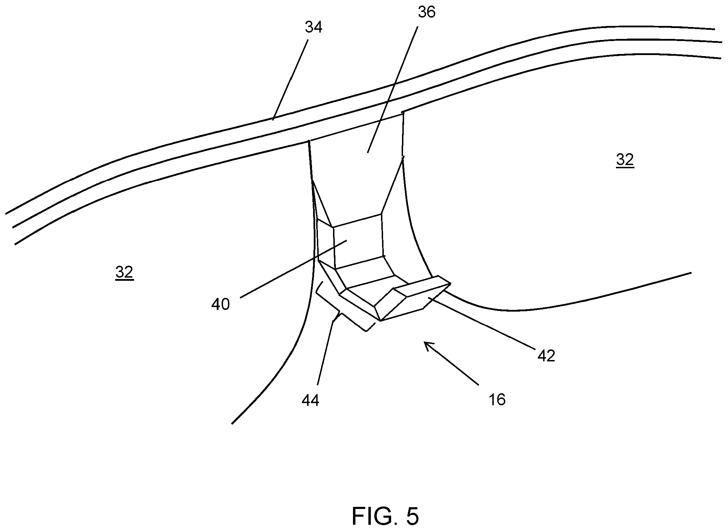

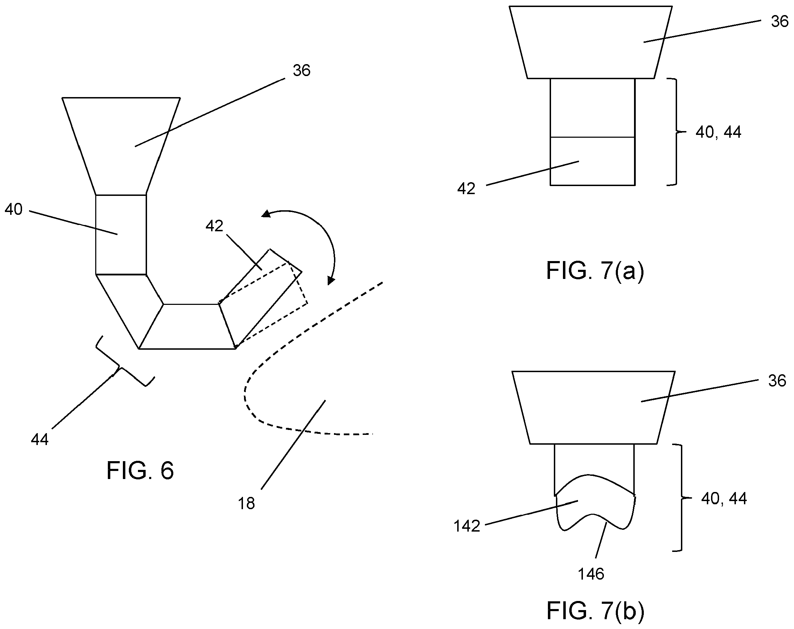

A frame support assembly comprising a nose bridge member 36 and the frame support member 16 is shown in FIGS. 4 and 5. The enlarged view in FIG. 5 illustrates one example configuration for the frame support member 16 have a vertically oriented portion 40 connected to an angled nose-engaging portion 42 via a contoured central portion 44. In this example, the central portion 44 includes a pair of segments angled with respect to each other to position the nose-engaging portion 42 behind the nose bridge 36 and vertical portion 40 as best seen in FIG. 6.

For reasons of comfort and/or to accommodate different users, the support member 16 can be constructed to include at least some inherent adjustability as shown in FIG. 6. In the example shown in FIG. 6, the nose engaging portion 42 is flexible relative to the central portion 44 to enable the angle of the nose engaging portion 42 to be adjusted to suit different nose shapes. It can be appreciated that any or all of the other portions of the frame support member 16 can include flexibility to allow for additional degrees of freedom of movement in adjusting the angle and position of the nose engaging portion 42. This allows the safety eyewear 10 to be supported above and away from underlying primary eyewear such as the prescription eyeglasses 12 shown in FIGS. 1 and 2.

FIG. 7(a) illustrates a front view of the frame support member 16 in one configuration in which the nose engaging portion 42 is substantially planar. However, as shown in FIG. 7(b) and according to an example not covered by the invention, a contoured nose engaging portion 142 can instead be provided which includes a contoured nose engaging surface 146 that generally follows the curvature of the upper surface of a user's nose 18. It can be appreciated that the particular contour 146 shown in FIG. 7(b) is purely illustrative and other contours can be used, including triangular, a many-sided "trough", etc.

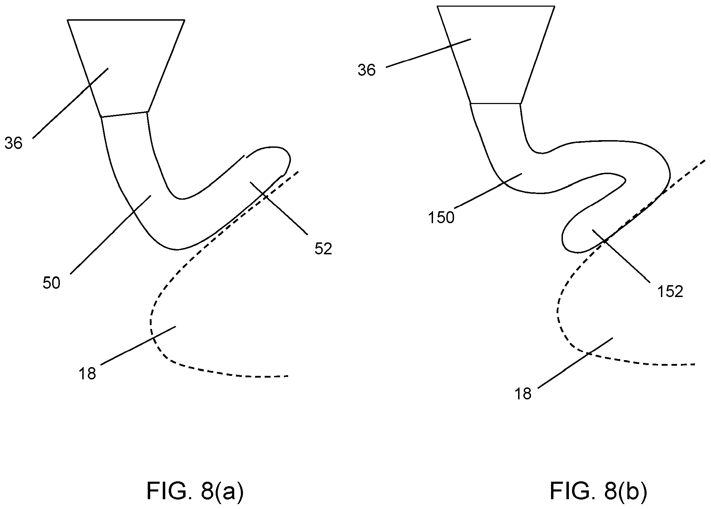

The multi-portion support member 16 shown in FIGS. 6 and 7 is also only one possible configuration. For example, as shown in FIG. 8(a), a single curved member 50 with a nose engaging surface 52 at its distal end can be used. As with other configurations, the member 50 can be provided with inherent flexibility by selecting a suitably flexible material for the support member 50. The generally "C" shape for the support member 16 is also only one possible configuration. For example, as shown in FIG. 8(b), any suitable shape that provides a nose engaging portion 152 that is positioned behind and below the nose bridge 36 can provide the same separation between the primary and secondary eyewear. However, it may be noted that the generally "C" shape can minimize the proportion of the frame support member 16 that could interfere with a nose bridge or nose pieces of the primary eyewear and can be sized to be able to fit underneath such a nose bridge.

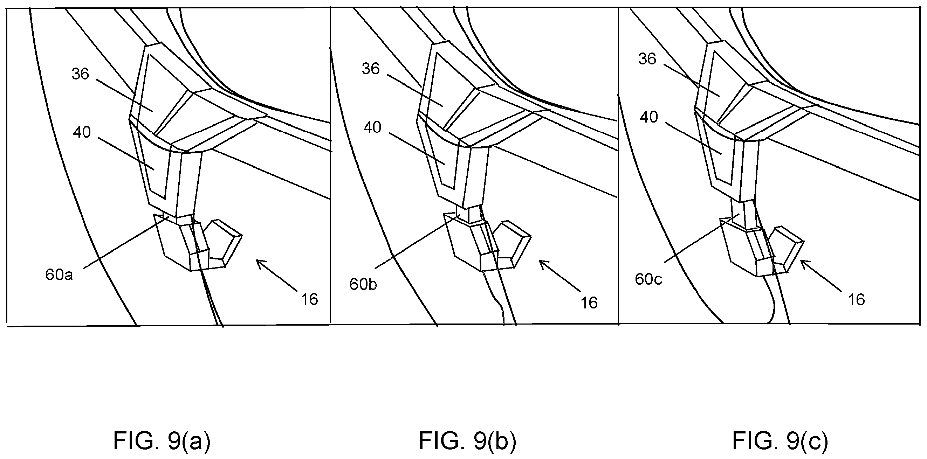

The adjustability of the frame support member 16 can also include extendibility, for example as shown in FIGS. 9(a) to 9(c). In the example shown in FIG. 9, the frame support member 16 and nose bridge 36 are connected to the vertically oriented portion 40, which can also be referred to as a frame engaging portion. The portion 40 is operatively connected to the remainder of the support assembly 16 via an extender 60. In this example, a frame support assembly is provided that includes the frame support member 16 adapted to include the extender 60. It can be appreciated that the vertical portion 40 (i.e. the frame engaging portion) can be constructed separately from the nose bridge 36 to enable the same safety eyewear 10 to be manufactured with or without the frame support assembly by providing a mechanism to couple the frame support assembly to the frame 10.

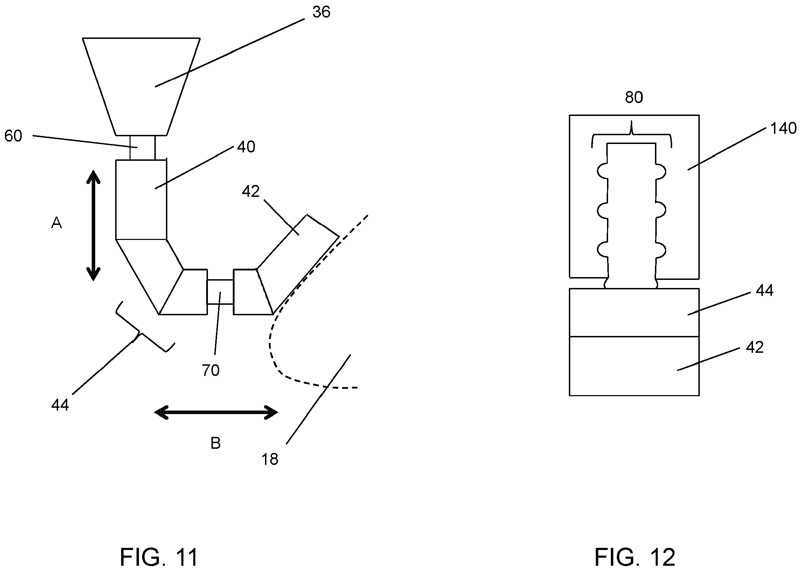

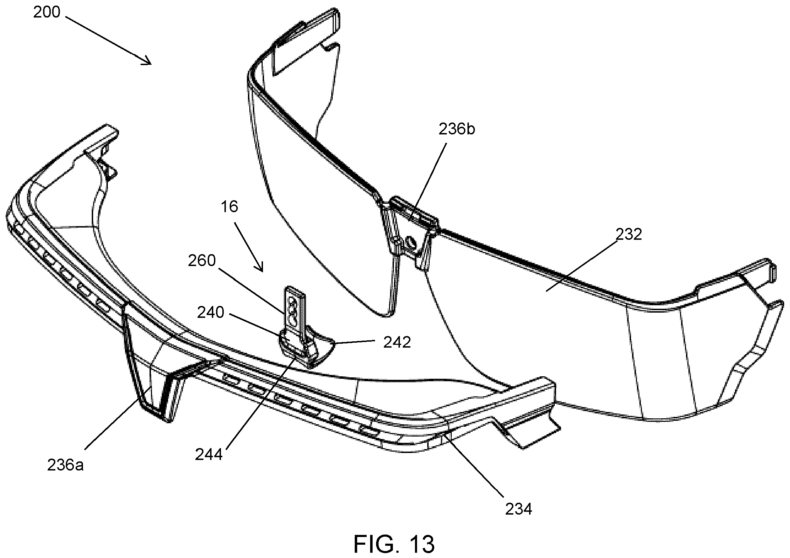

The extender 60 can be made extendible in various ways. For example, as shown in the figures, the extender 60 can be fixed to the frame support member 16 at one end and moveably connected to the vertical portion 40 (and nose bridge 36) at its other end. The moveable connection can be a ratchet-type mechanism 80 (see FIGS. 12 and 13), a frictional engagement, or using any other suitable adjustment means in order to provide a plurality of positions. The plurality of positions correspond to a plurality of distances between the nose engaging member 42 and the nose bridge 36 and frame 34. In this way, the extender 60 can be used to adjust the vertical separation between the primary and secondary eyewear. FIG. 9(a) illustrates the extender 60a in a first position, FIG. 9(b) illustrates the extender 60b in a second position, and FIG. 9(c) illustrates the extender 60c in a third position. It can be appreciated that the positions shown in FIGS. 9(a)-9(c) can represent a limited number of discrete positions or positions that are possible in a continuum, e.g., with a frictional connection allowing for a multitude of positions. That is, the provision of three discrete positions is illustrative only.

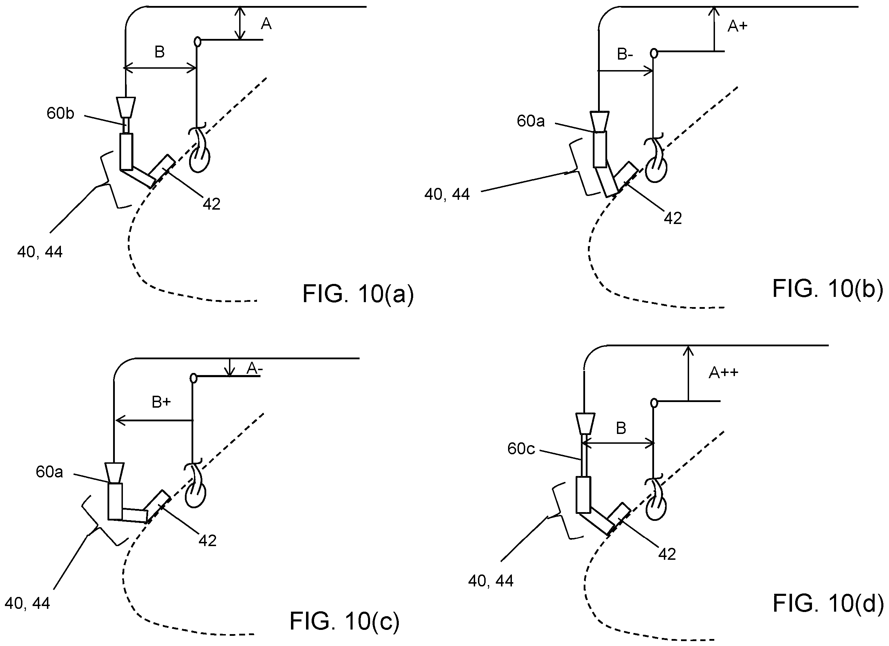

FIGS. 10(a) to 10(d) illustrate how the extender 60 and inherent flexibility in at least a portion of the frame support assembly 16 enables adjustability to suit many users. FIG. 10(a) illustrates a first configuration as a reference point, in which the extender 60b is in the second position shown in FIG. 9(b). In this first configuration, a vertical separation A, and a horizontal separation B are provided between the primary and secondary eyewear. FIG. 10(b) illustrates that by flexing the frame support member 16, an increased vertical separation A+ and decreased horizontal separation B- can be achieved, even with the extender 60a being in the first position shown in FIG. 9(a). As such, the combination of flexibility and extendibility enables adjustability in both directions.

In another example shown in FIG. 10(c), the frame support member 16 is flexed to push the nose engaging portion 42 further back, while raising it relative to the other portions 40, 44. This provides an increased horizontal separation B+, and decreased vertical separation A- when compared to FIG. 10(a). In a further example shown in FIG. 10(d), the same horizontal separation B as shown in FIG. 10(a) is provided with a further increased vertical separation A++ by using the extender 60c in the third position. It can be appreciated that the examples shown in FIG. 10 are illustrative and that many additional configurations are possible through a combination of flexibility and/or extendibility of the frame support member 16 and/or the overall frame support assembly.

The multiple directions of adjustability exemplified herein can also be provided using multiple extenders 60, 70 as shown in FIG. 11. In this example, a second horizontally oriented extender 70 connects the central portion 46 to the nose engaging portion 42 of the frame support member 16. This allows the nose engaging portion 42 to be adjusted rearwardly of the nose bridge 36 as well as downwardly therefrom. It can be appreciated that the second extender 70 can be used with substantially rigid portions 40, 42, 44, or can be used with at least one flexible portion, e.g., the nose engaging portion 42 to allow for different angular orientations thereof.

FIGS. 13 to 21 illustrate an alternative adjustment assembly using an extendible frame support member 16. In this example, the frame support member 16 includes an extender 260 that is interposed between a frame 234 and a lens 232 of an eyewear assembly 200. It can be appreciated that the assembly 200 shown in FIG. 13 would also typically include a pair of arms for engaging a user's ears.

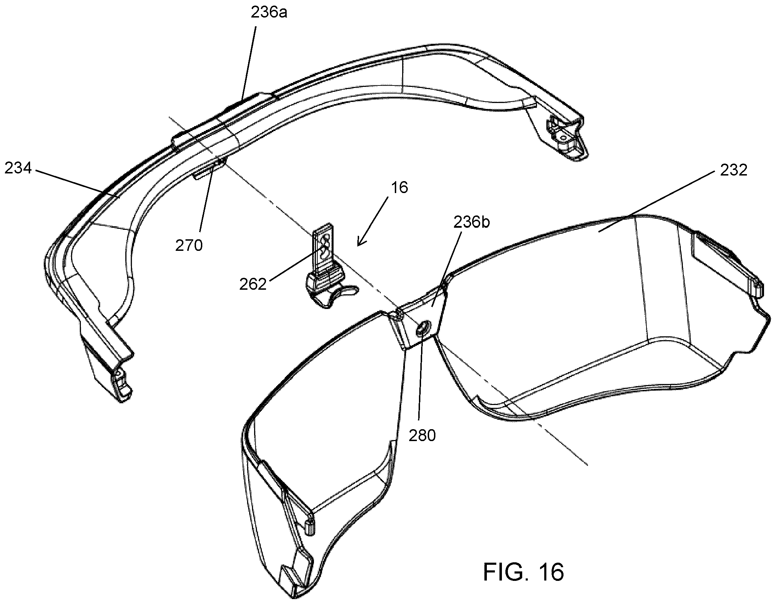

Turning first to FIG. 13, the frame 234 includes a centrally positioned bridge portion 236a that connects through the frame support member 16 to a bridge portion 236b of the lens 232. This is shown from the opposite perspective view in FIG. 16. The support member 16 includes an extender 260 that is connected to a vertical portion 204 that in turn connects to a nose engaging portion 242 via a central portion 244, similar to the above embodiments.

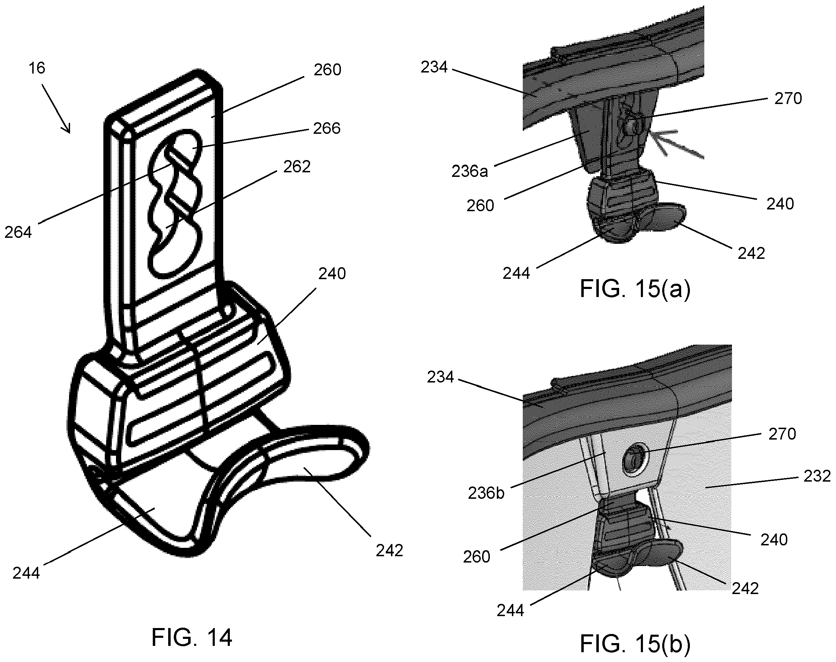

FIG. 14 provides a more detailed view of the support member 16 in isolation. The support member 16 includes a contoured aperture 262 in the extender 260. The contoured aperture 262 includes a number of peaks 264 and valleys 266 that define a series of settings or positions such that a member that is sized substantially similar to the area between opposing valleys 266, for each setting, can be secured into and thus be positioned at that setting. As can be seen in FIG. 14, opposing valleys 266 of the interior sidewalls of the extender 260 each provide a generally circular area whose distance can be made to correspond substantially to the diameter of a circular member extending therethrough. The peaks 264 imposed between the areas at each position provide restricted areas along the aperture 262 such that any movement in the vertical direction would require additional effort to move the member through the restricted or otherwise narrowed sections between each setting to create a vertical adjustment mechanism for adjusting the position of the support member 16 relative to the frame 234 and lens 232 That is, the opposing peaks 264 are closer to each other than the opposing valleys 266 to resist free movement of the extendable attachment portion 260 relative to the frame 234.

In the view shown in FIG. 15(a), it can be seen that the bridge portion 236a can include a post 270 that acts as the aforementioned circular member extending through the aperture 262. In this way, the extender 260 can adjust the vertical positioning of the nose engaging member 242 relative to the eyewear assembly 200. The post 270 can have a relatively larger head with a central split to permit the post 270 to snap into engagement with the body of the post 270 seated within the aperture 262 as shown in FIG. 15(a). As illustrated in FIG. 15(b), the head portion of the post 270 can also snap into a hole 280 in the bridge portion 236b of the lens 232 to complete the assembly (see also FIG. 16).

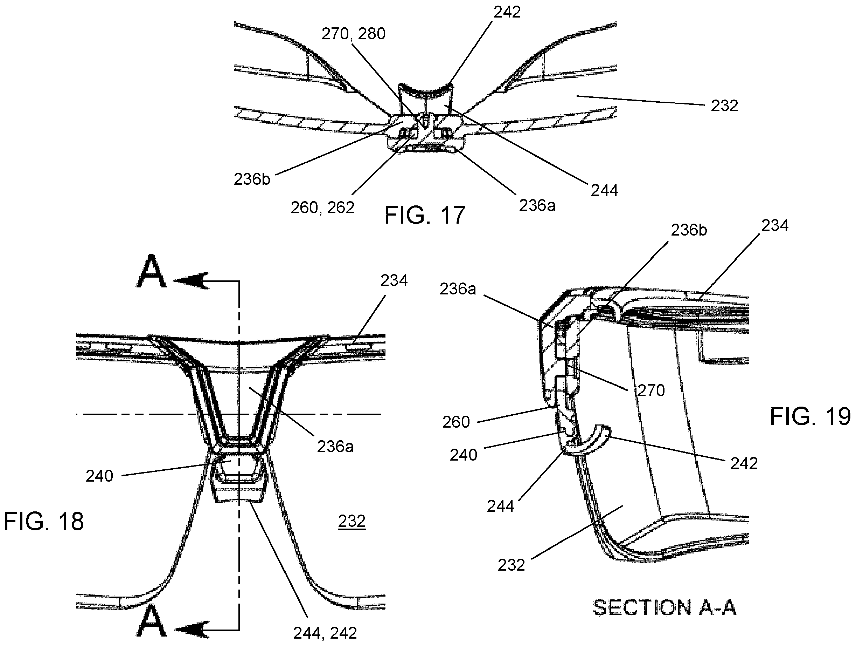

As illustrated in FIGS. 13 and 16, the bridge portion 236b of the lens 232 can be slotted or otherwise configured to constrain the support member 16 to vertical movements, e.g., to inhibit rotation of the support member 16 relative to the eyewear assembly 200. This can also be seen in the cross-sectional view of FIG. 17 wherein when the post 270 is snapped into the hole 280 in the lens 232, the extender 260 is seated within the recessed area of the bridge portion 236b of the lens 232 with the bridge portion 236a of the frame 234 secured against the front of the lens 232. This positions the post 270 in a fixed vertical location such that vertical movement of the frame support member 16 adjusts the length of the extender 260 beyond the lowermost edge of the bridge portion 236a of the frame 234.

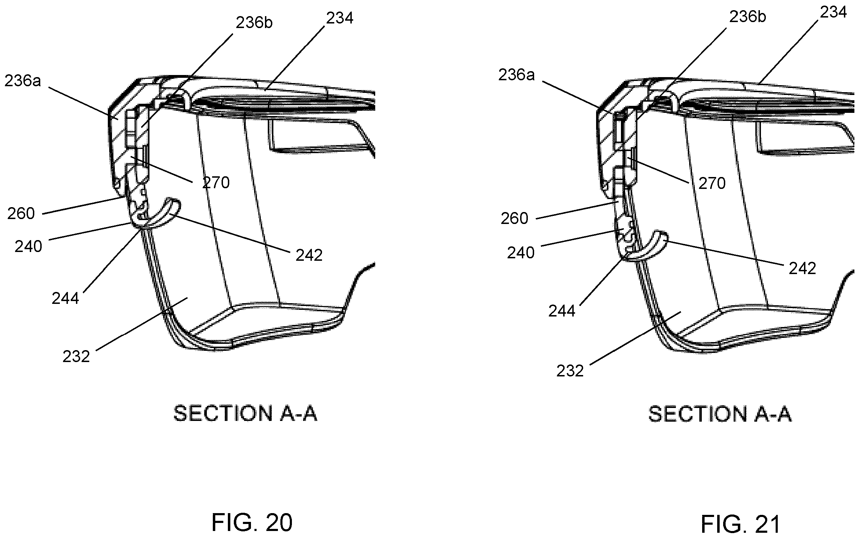

FIG. 18 provides a front view of the central area of eyewear assembly 200. Section A-A from FIG. 18 is shown in three different adjustable positions in FIGS. 19-21. In FIG. 19, a middle position is shown with the post 270 being positioned in a middle position of the contoured aperture 262. FIG. 20 illustrates an upper position with the post 270 being positioned in an uppermost position of the contoured aperture 262 to therefore place the nose engaging portion 242 closer to the frame 234 than the position shown in FIG. 19. FIG. 21 illustrates a lower position with the post 270 being positioned in a lowermost position of the contoured aperture 262 to therefore place the nose engaging portion 242 further from the frame 234 than the position shown in FIG. 19.

It can be appreciated that while the contoured aperture 262 (seen best in FIG. 14) is configured to have three positions, the aperture 262 can also include more or fewer positions by having more or fewer areas that are sized substantially the same as the post 270. It can also be appreciated that the extender 260 can be applied to any nose piece or support member to provide vertical adjustability thereof, and thus is not restricted to being used with the frame support members 16 described herein.

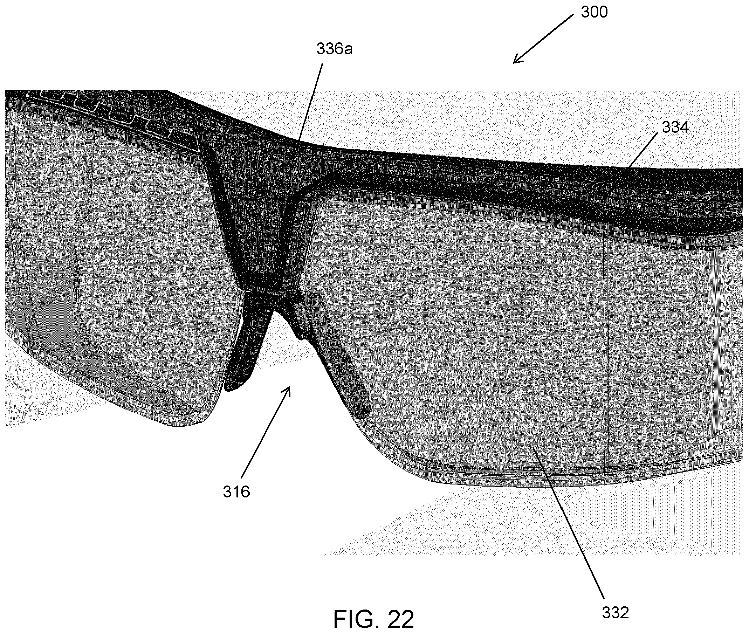

FIGS. 22-27 illustrate an adjustment assembly similar to that shown in FIGS. 13-21, with an alternative extendible frame support member 316. In this example, the frame support member 316 is interposed between a frame 334 and a lens 332 of an eyewear assembly 300. It can be appreciated that the eyewear assembly 300 shown in FIG. 22 would also typically include a pair of arms for engaging a user's ears and/or head.

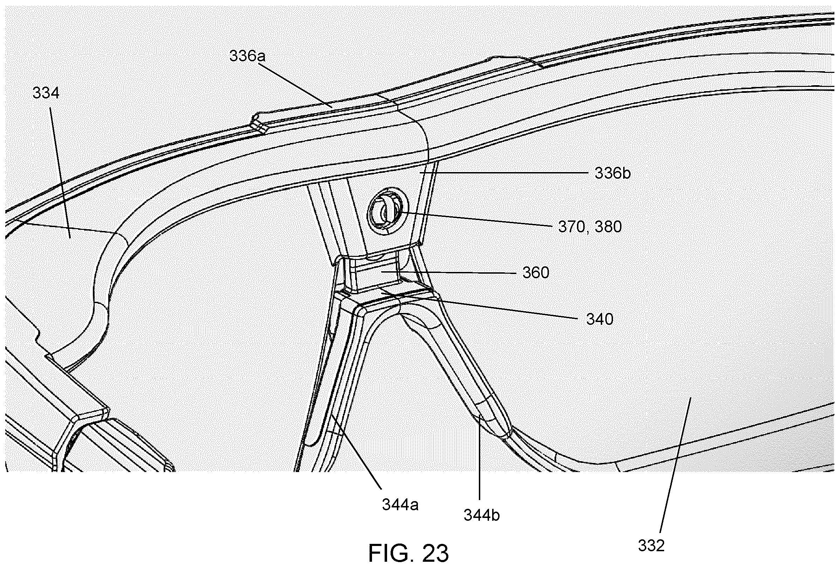

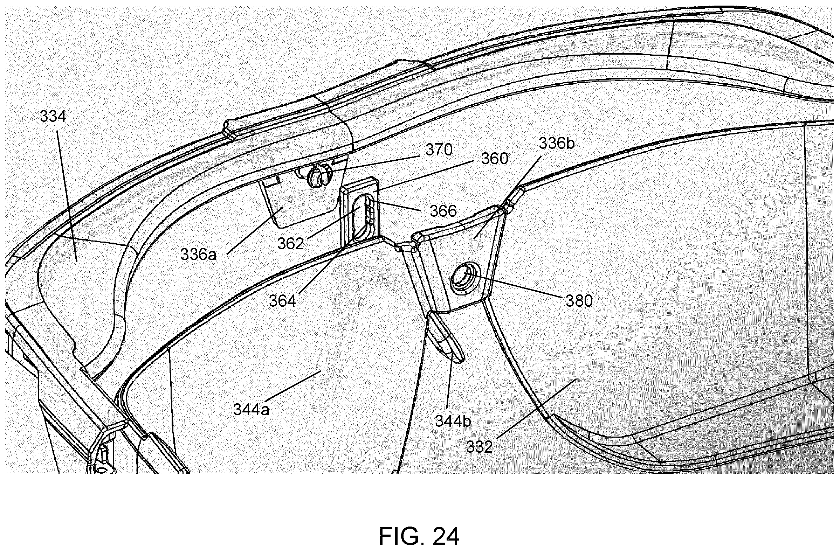

Turning first to FIGS. 22 and 23, the frame 334 includes a centrally positioned bridge portion 336a that connects through an extender 360 of the frame support member 316 to a bridge portion 336b of the lens 232. Referring additionally to FIG. 24, a post 370 extends from the bridge portion 336a of the frame 334 through a hole 380 in the bridge portion 336b of the lens 332, similar to the embodiment in FIGS. 13-21. In this example, the post 370 is slotted to permit separation and resilience in order to provide a "snap-in" fit into the hole 380 as illustrated in FIG. 23. The extender 360 is connected to a nosepiece 340. The nosepiece 340 in this example forks into a pair of nose engaging pads 344a, 344b, each sized and positioned to rest against opposing sides of a user's nose. It can be appreciated that the nosepiece 340 and nose engaging pads 344a, 344b can be made from a rigid material, or can be made of a flexible material, similar to the embodiments described above, in order to permit adjustability of the eyewear assembly 300 relative to a pair of prescription eyewear 12 in an OTG application. This flexibility and adjustability can be provided in any one or more directions, for example in the fore and aft directions as well as to pinch together or separate the pads 344a, 334b.

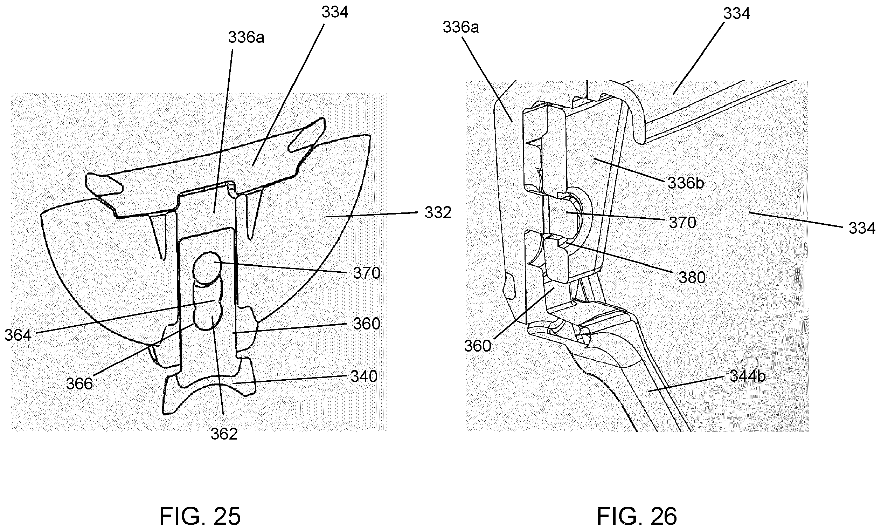

As shown in greater detail in FIGS. 24 and 25, the support member 316 includes a contoured aperture 362 in the extender 360. The contoured aperture 362 includes a number of peaks 364 and valleys 366 that define a series of settings or positions such that a member that is sized substantially similar to the area between opposing valleys 366, for each setting, can be secured into and thus be positioned at that setting. As can be seen in FIG. 24, opposing valleys 366 of the interior sidewalls of the extender 360 each provide a generally curved or circular area whose distance can be made to correspond substantially to the diameter of a circular member extending therethrough, namely the post 370 in this example. The peaks 364 imposed between the areas at each position provide restricted areas along the aperture 362 such that any movement in the vertical direction would require additional effort to move the post 370 through the restricted or otherwise narrowed sections between each setting to create a vertical adjustment mechanism for adjusting the position of the support member 316 relative to the frame 334 and lens 332 That is, the opposing peaks 364 are closer to each other than the opposing valleys 366 to resist free movement of the extendable attachment portion 360 relative to the frame 334 during normal use.

Turning to FIGS. 25 and 26, these sectioned illustrate that the extender 360 is slidable between the bridge portions 336a, 336b but restricted in its movement and positioning by the post 370 that snaps into the hole 380. This not only restricts the movement and positioning, but also retains the support member 316 with the eyewear assembly 300.

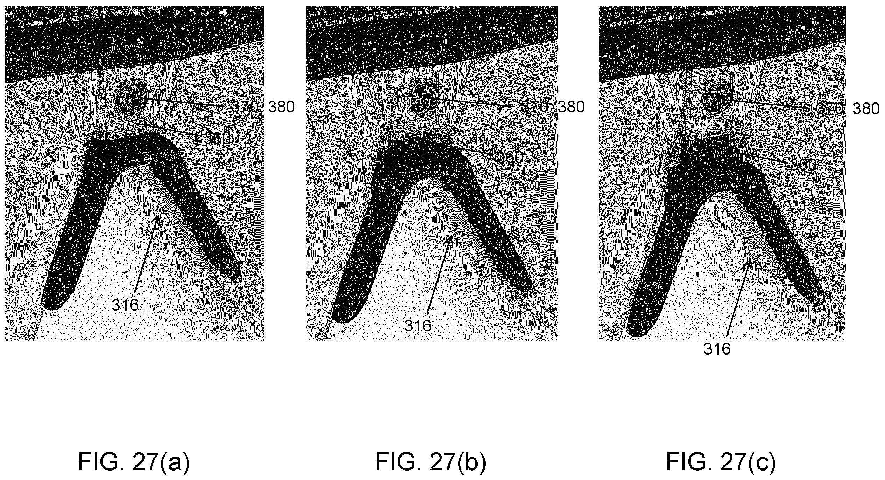

The series of views shown in FIGS. 27(a), 27(b), and 27(c) illustrate the adjustability of the support member 316 relative to the lens 332, in this example a low position in FIG. 27(a), a mid-position in FIG. 27(b), and a high position in FIG. 27(c).

It can therefore be appreciated from the embodiment shown in FIGS. 22-27 that the alternative adjustment assembly shown in FIGS. 13-21 can be applied to various types of frame support members 16, 316, including those with different types of nose pieces and nose engaging members.

For simplicity and clarity of illustration, where considered appropriate, reference numerals may be repeated among the figures to indicate corresponding or analogous elements. In addition, numerous specific details are set forth in order to provide a thorough understanding of the examples described herein. However, it will be understood by those of ordinary skill in the art that the examples described herein may be practiced without these specific details. In other instances, well-known methods, procedures and components have not been described in detail so as not to obscure the examples described herein. Also, the description is not to be considered as limiting the scope of the examples described herein.

It will be appreciated that the examples and corresponding diagrams used herein are for illustrative purposes only. Different configurations and terminology can be used without departing from the principles expressed herein. For instance, components and modules can be added, deleted, modified, or arranged with differing connections without departing from these principles.

Although the above principles have been described with reference to certain specific examples, various modifications thereof will be apparent to those skilled in the art as outlined in the appended claims.

## Claims

1. A frame support member (16) for eyewear (10) comprising a frame (34), the frame (34) of the eyewear (10) comprising a nose bridge (36), the frame support member (16) comprising:
an attachment portion (40) at a first end thereof for coupling the frame support member (16) to the nose bridge (36) of the eyewear; and
a nose engaging portion (42) at a second end thereof, the nose engaging portion (42) being positioned to engage an upper surface of a user's nose (18);
wherein the frame support member (16) is shaped such that it extends downwardly and rearwardly to engage the user's nose, and configured to position the nose engaging portion (42) below and behind the nose bridge (36) to support the eyewear on the user above and forward of the user's nose according to the positioning of the nose engaging portion;
**characterized by** the nose engaging portion being substantially planar and configured to engage the user's nose along a center line of the nose.

2. The frame support member of claim 1, further comprising at least one extender (60, 70) for adjusting the positioning of the nose engaging portion (42).

3. The frame support member of claim 2, wherein the at least one extender comprises a vertically oriented extender (60).

4. The frame support member of claim 2, wherein the at least one extender comprises a horizontally oriented extender (70).

5. The frame support member of claim 1, further comprising at least one flexible portion for adjusting the positioning of the nose engaging portion (42).

6. The frame support member of claim 5, wherein the at least one flexible portion enables the nose engaging portion (42) to be angularly adjusted.

7. Eyewear comprising:
a frame for supporting at least one lens;
a nose bridge for supporting the frame on a user; and
a frame support member according to any one of claims 1 to 6.

8. The eyewear of claim 7, wherein the eyewear (10) is secondary eyewear (10) worn over primary eyewear (12), the frame support member (16) being configured to provide separation between the secondary eyewear (10) and the primary eyewear (12) when both eyewear are supported on the user's nose (18).

9. The eyewear of claim 7, wherein the secondary eyewear is safety eyewear (32).

10. The eyewear of claim 7, further comprising a pair of arms coupled to the frame.

11. The eyewear of claim 10, wherein:
the frame and nose bridge are formed to support a pair of lenses;
the pair of arms are pivotally attached to the frame;
the frame support member is coupled to the nose bridge via an extender; and
at least a portion of the frame support member has flexibility.

12. The frame support member of claim 1, wherein the nose engaging portion (42) is configured to engage the user's nose (18) along a longitudinal axis thereof.

13. The frame support member of claim 1, wherein the attachment portion (40) further comprises a contoured aperture for receiving a post extending from the nose bridge portion (36) or the lens of the eyewear, the post engaging the contoured aperture to provide a plurality of vertical positions relative to the frame.

14. The frame support member of claim 2, wherein the at least one extender comprises a vertically oriented extender (60) and a horizontally oriented extender (70).

## Patentansprüche

1. Rahmenstützglied (16) für eine Brille (10) mit einem Rahmen (34), wobei der Rahmen (34) der Brille (10) einen Nasensteg (36) aufweist, wobei das Rahmenstützglied (16) aufweist:
einen Befestigungsbereich (40) an einem ersten Ende desselben zum Verbinden des Rahmenstützglieds (16) mit dem Nasensteg (36) der Brille; und
einen Nasenanlagebereich (42) an einem zweiten Ende desselben, wobei der Nasenanlagebereich (42) zur Anlage an der Oberseite der Nase (18) des Benutzers positioniert ist;
wobei das Rahmenstützglied (16) derart geformt ist, dass es sich nach unten und hinten erstreckt, um an der Nase des Benutzers anzuliegen, und dazu ausgebildet ist, den Nasenanlagebereich (43) unter und hinter dem Nasensteg (36) zu positionieren, um die Brille gemäß der Positionierung des Nasenanlagebereichs oberhalb und vor seiner Nase des Benutzers zu stützen;
**dadurch gekennzeichnet, dass** der Nasenanlagebereich im Wesentlichen planar ist und dazu ausgebildet ist, an der Nase des Benutzers entlang einer Mittellinie der Nase anzuliegen.

2. Rahmenstützglied nach Anspruch 1, ferner mit mindestens einer Verlängerung (60, 70) zum Einstelen der Positionierung des Nasenanlagebereichs (42).

3. Rahmenstützglied nach Anspruch 2, bei welchem die mindestens eine Verlängerung eine vertikal gerichtete Verlängerung (60) aufweist.

4. Rahmenstützglied nach Anspruch 2, bei welchem die mindestens eine Verlängerung eine horizontal gerichtete Verlängerung (70) aufweist.

5. Rahmenstützglied nach Anspruch 1, ferner mit mindestens einem flexiblen Bereich zum Einstellen der Positionierung des Nasenanlagebereichs (42) aufweist.

6. Rahmenstützglied nach Anspruch 5, bei welchem der mindestens eine flexible Bereich ein winkliges Einstellen des Nasenanlagebereichs (42) ermöglicht.

7. Brille mit:
einem Rahmen zum Stützen mindestens einer Linse;
einem Nasensteg zum Stützen des Rahmens an einem Benutzer; und
einem Rahmenstützglied nach einem der Ansprüche 1 bis 6.

8. Brille nach Anspruch 7, bei welcher die Brille (10) eine über einer primären Brille (12) getragene sekundäre Brille (10) ist, wobei das Rahmenstützglied (16) dazu ausgebildet ist, eine Trennung zwischen der sekundären Brille (10) und der primären Brille (12) zu schaffen, wenn beide Brillen auf der Nase (18) des Benutzers gestützt sind.

9. Brille nach Anspruch 7, bei welcher die sekundäre Brille eine Sicherheitsbrille (32) ist.

10. Brille nach Anspruch 7, ferner mit zwei mit dem Rahmen verbundenen Armen.

11. Brille nach Anspruch 10, bei welcher:
der Rahmen und der Nasensteg zum Stützen zweier Linsen geformt sind;
die beiden Arme schwenkbar an dem Rahmen angebracht sind;
das Rahmenstützglied mit dem Nasensteg über eine Verlängerung verbunden ist; und
zumindest ein Bereich des Rahmenstützglieds Flexibilität aufweist.

12. Rahmenstützglied nach Anspruch 1, bei welchem der Nasenanlagebereich (42) dazu ausgebildet ist, an der Nase (18) des Benutzers entlang einer Längsachse derselben anliegt.

13. Rahmenstützglied nach Anspruch 1, bei welchem der Befestigungsbereich (40) ferner eine konturierte Öffnung zum Aufnehmen eines sich von dem Nasenstegbereich (36) oder der Linse der Brille erstreckenden Stifts aufweist, wobei der Stift in die konturierte Öffnung eingreift, um mehrere vertikale Positionen relativ zum Rahmen bereitzustellen.

14. Rahmenstützglied nach Anspruch 2, bei welchem die mindestens eine Verlängerung eine vertikal ausgerichtete Erweiterung (60) und eine horizontale Erweiterung (70) aufweist.

## Revendications

1. Organe de support de monture (16) pour des lunettes (10) comprenant une monture (34), la monture (34) des lunettes (10) comprenant un pont de nez (36), l'organe de support de monture (16) comprenant :
une partie de fixation (40) à une première extrémité de celui-ci pour coupler l'organe de support de monture (16) au pont de nez (36) des lunettes ; et
une partie de contact avec le nez (42) à une deuxième extrémité de celui-ci, la partie de contact avec le nez (42) étant positionnée pour entrer en contact avec une surface supérieure du nez d'un utilisateur (18) ;
dans lequel l'organe de support de monture (16) est façonné de manière à s'étendre vers le bas et vers l'arrière pour entrer en contact avec le nez de l'utilisateur, et configuré pour positionner la partie de contact avec le nez (42) au-dessous et à l'arrière du pont de nez (36) pour supporter les lunettes sur l'utilisateur au-dessus et à l'avant du nez de l'utilisateur selon le positionnement de la partie de contact avec le nez ;
**caractérisé en ce que** la partie de contact avec le nez est sensiblement plane et configurée pour entrer en contact avec le nez de l'utilisateur le long d'une ligne centrale du nez.

2. Organe de support de monture selon la revendication 1, comprenant en outre au moins un prolongateur (60, 70) pour ajuster le positionnement de la partie de contact avec le nez (42).

3. Organe de support de monture selon la revendication 2, dans lequel l'au moins un prolongateur comprend un prolongateur orienté verticalement (60).

4. Organe de support de monture selon la revendication 2, dans lequel l'au moins un prolongateur comprend un prolongateur orienté horizontalement (70).

5. Organe de support de monture selon la revendication 1, comprenant en outre au moins une partie souple pour ajuster le positionnement de la partie de contact avec le nez (42).

6. Organe de support de monture selon la revendication 5, dans lequel l'au moins une partie souple permet le réglage angulaire de la partie de contact avec le nez (42).

7. Lunettes comprenant :
une monture pour supporter au moins un verre ;
un pont de nez pour supporter la monture sur un utilisateur ; et
un organe de support de monture selon l'une des revendications 1 à 6.

8. Lunettes selon la revendication 7, dans lesquelles les lunettes (10) sont des lunettes secondaires (10) portées sur des lunettes primaires (12), l'organe de support de monture (16) étant configuré pour fournir une séparation entre les lunettes secondaires (10) et les lunettes primaires (12) lorsque les deux lunettes sont supportées sur le nez de l'utilisateur (18).

9. Lunettes selon la revendication 7, dans lesquelles les lunettes secondaires sont des lunettes de sécurité (32).

10. Lunettes selon la revendication 7, comprenant en outre une paire de branches couplées à la monture.

11. Lunettes selon la revendication 10, dans lesquelles :
la monture et le pont de nez sont formés pour supporter une paire de verres ;
la paire de branches sont fixées à pivotement à la monture ;
l'organe de support de monture est couplé au pont de nez par un prolongateur ; et au moins une partie de l'organe de support de monture est souple.

12. Organe de support de monture selon la revendication 1, dans lequel la partie de contact avec le nez (42) est configurée pour entrer en contact avec le nez de l'utilisateur (18) le long d'un axe longitudinal de celui-ci.

13. Organe de support de monture selon la revendication 1, dans lequel la partie de fixation (40) comprend en outre une ouverture profilée pour recevoir un montant s'étendant à partir de la partie de contact avec le nez (36) ou du verre des lunettes, le montant entrant en contact avec l'ouverture profilée pour fournir une pluralité de positions verticales par rapport à la monture.

14. Organe de support de monture selon la revendication 2, dans lequel l'au moins un prolongateur comprend un prolongateur orienté verticalement (60) et un prolongateur orienté horizontalement (70).
